# EUROPEAN PATENT SPECIFICATION

(11) **EP 2 349 154 B1**
(45) Date of publication and mention of the grant of the patent: **27.08.2014**
(21) Application number: 09744278.4
(22) Date of filing: 23.10.2009
(51) Int. Cl.: A61F 13/06, A61F 13/10, A61F 13/00

(54) **WOUND DRESSING**
WUNDVERBAND
PANSEMENT

(30) Priority: 24.10.2008 US 108397 P
(43) Date of publication of application: 03.08.2011
(73) Proprietor: 3M Innovative Properties Company, Saint Paul, MN 55133-3427 (US)
(72) Inventor: HOLM, David R.,, Saint Paul, Minnesota 55133-3427 (US); Richard Lee Jacobson, Saint Paul, Minnesota 55133-3472 (US)
(74) Representative: Vossius & Partner
(86) International application number: PCT/US2009/061791
(87) International publication number: WO 2010/048480

(56) References cited:
- DE-A1-102005 009 634
- GB-A- 2 131 299
- US-A- 6 121 508
- US-A1- 2004 127 832

## Description

### Field of the Invention

This disclosure relates to wound dressings, and more particularly to a wound dressing and a support delivery system designed to conform to highly contoured areas of the body.

### Background of the Invention

Wound dressings using an absorbent central area surrounded by a larger adhesive film are known. For example, U.S. Patent No. 5,738,642 (the '642 patent) shows a wound dressing and delivery system comprising a thick absorbent pad placed in the center of a thin backing. A carrier frame surrounds the perimeter of the wound dressing, providing support (e.g. rigidity) to the backing to facilitate handling of the dressing during application to a wound. Such dressings are often applied to relatively flat areas of the body, such as the chest or abdomen.

### Summary of the Invention

The present disclosure provides a wound dressing according to the claims. The wound dressing has a backing material surrounding an absorbent pad, and delivery system that facilitates the handling and application of the wound dressing. The wound dressing is readily handled and applied with one hand, which frees up the other hand to help position the patient or parts of the patient to ensure better application of the wound dressing. The wound dressing is constructed so as to have improved adhesion to highly contoured areas of the body, such as a toe, finger or heel, and to permit easy application to these surfaces.

In certain embodiments, the wound dressing and delivery system comprise an adhesive layer on a first side of a backing layer, with absorbent pad or other material applied or attached to the adhesive layer. A dressing support layer is positioned on a second side of the backing layer, and is configured to deliver the wound dressing to a wound. The dressing support layer further comprises a tab, wherein the tab overlaps at least a portion of the absorbent pad on the opposite side of the backing layer of the wound dressing.

These and various other advantages and features characterizing the invention are pointed out with particularity in the claims annexed hereto and forming a part hereof. However, for a better understanding of the invention, its advantages, and objects obtained by its use, reference should be made to the accompanying drawings and descriptive matter, in which embodiments of the invention are illustrated and described.

### Brief Description of the Drawings

The invention will be further described with reference to the drawings, wherein corresponding reference characters indicate corresponding parts throughout the several views, and wherein:
FIG. 1 is a top view of a wound dressing according to one embodiment of the present disclosure.
FIG. 2 is side view of the wound dressing of FIG. 1.
FIG. 3 is a top view of a wound dressing according to a further embodiment of the present disclosure.
FIG. 4 is a side view of the wound dressing of FIG. 3.
FIG. 5 is a top view of a wound dressing in a further embodiment of the present disclosure.
FIG. 6 is a top view of a wound dressing in a further embodiment of the present disclosure.
FIGs. 7a-c depict the wound dressing of FIG. 3 being applied to a patient.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the claims.

### Detailed Description

The present disclosure is directed to a wound dressing and delivery system, as well as to methods of positioning and applying the wound dressing to a patient, especially with one hand. The wound dressing and delivery system are particularly suited for application over a highly contoured surface of a patient, such as a finger, toe or heel. The wound dressing permits an absorbent material to be placed against the wound, while the delivery system aids in positioning the wound dressing on the body over the wound site, and forming a seal around the wound.

In some embodiments, the wound dressing comprises an absorbent pad, a backing layer, and an adhesive layer on the backing layer facing the absorbent pad. The adhesive layer and backing layer form a perimeter around the absorbent pad and hold the absorbent pad in place on a wound. The perimeter formed by the adhesive layer and backing layer keeps the absorbent pad properly positioned, and may also help maintain a sterile environment around the wound.

The delivery system described herein provides an easy and effective way to position and apply the wound dressing to a patient with one hand and is particularly well suited for application of a wound dressing to a highly contoured area of the body such as to the fingers, toes, or a heel. The wound dressings described herein are generally comprised of a backing layer having an adhesive layer on one side of the backing layer, and an absorbent pad on the adhesive layer. A support layer is releasably adhered to the backing layer on the side opposite the adhesive layer. The support layer includes a tab that overlaps a portion of the absorbent pad located on the opposite side of the backing. In some embodiments, the tab is covered with a bond block material, or is free of adhesive in order to permit easy handling of the tab. The wound dressing may further comprise a release liner to protect the adhesive until the dressing is ready for use.

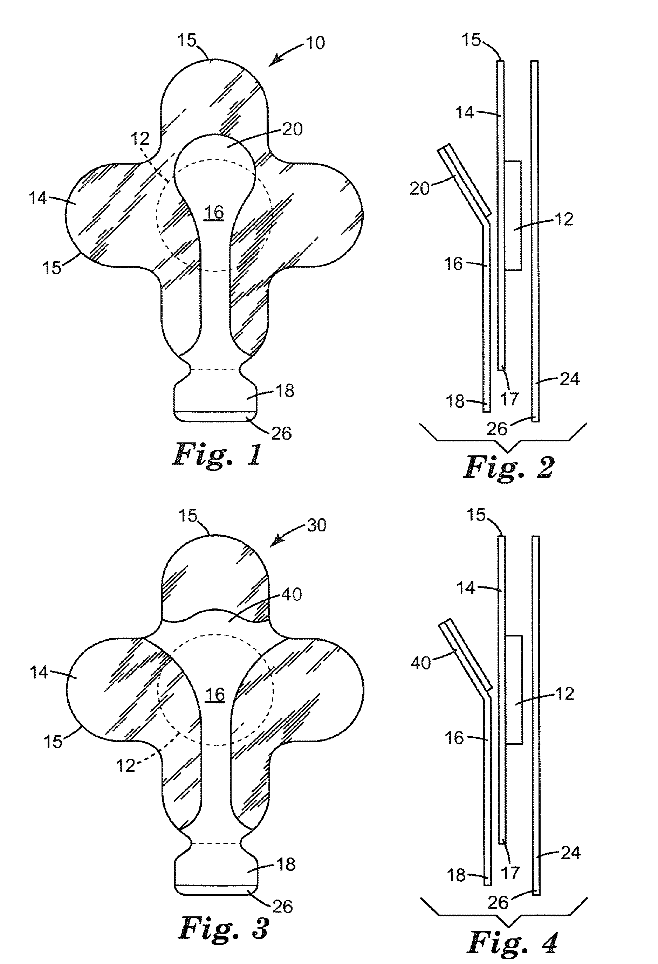

Turning now to the figures, FIGs. 1 and 2, show an example of a wound dressing and delivery system in accordance with one embodiment of the disclosure. FIG. 1 is a top view of the wound dressing, and FIG. 2 is a side view of the wound dressing of FIG. 1. The wound dressing 10 includes a centrally located absorbent pad 12, covered by an adhesive layer 17 on one side of a backing layer 14 that extends out to the perimeter 15 of the wound dressing 10. The backing layer 14, as described in further detail below, may be made of a polyurethane film substrate that is laminated to a non-woven, woven, or knitted material.

At least one dressing support layer 16 is positioned over the wound dressing 10. In the embodiment shown in FIG. 1, the dressing support layer 16 has a substantially linear configuration, with a tab portion 20 at one end, wherein the tab portion 20 substantially overlaps the position of the absorbent pad 12, and an end portion 18, at the opposing end of the support layer 16. The support layer 16 can be a single piece of material, such as a polymeric film, or can be two or more distinct pieces. The support layer 16 shown in FIGS. 1 and 2 is a single piece of material which is at least partially secured to the backing layer 14, for example, by heat seal bonding or with the use of an adhesive. Methods of heat sealing can be found, for example, in U.S. Patent No. 5,928,972.

In an embodiment, the tab portion is not secured to the backing 14 by use of a bond block material or liner to facilitate handling the wound dressing 10 by the tab 20. Examples of suitable bond block materials are described in U.S. Patent No. 7,442,849 (Heinecke).

The wound dressing 10 typically includes a carrier film 24 to protect the adhesive layer 17, until the wound dressing is ready for use. To facilitate removal, the carrier film 24 may have a tab 26 which overhangs the end portion 18 of the support layer 16. The carrier film 24 covers the surface of the wound dressing applied to the patient, generally making contact with the absorbent pad 12 and the periphery of the adhesive layer 17. The carrier film 24 remains attached to wound dressing 10 until a user is ready to apply the dressing. The carrier film 24 may be a single piece or multiple piece release liner, and may be part of or laminated to the package (not shown) containing the dressing, or merely enclosed along with the dressing within the package. The carrier film 24 keeps the adhesive clean during storage and shipping of the wound dressing 10.

The wound dressing 10 is typically applied to a patient by first cleaning the wound and making sure the area around the wound is ready to receive a dressing. The carrier film 24 is then removed from the dressing, for example, by grasping tab 26 and peeling away the carrier film 24, exposing the bottom of the absorbent pad 12 and the perimeter of the adhesive layer 17 on the backing layer 14. As the wound dressing 10 is held by tab 20, the center of the absorbent pad 12 is brought in contact with the wound, and then the edges of the dressing 10 are gently pressed against the patient, thereby bringing the exposed adhesive perimeter of the backing layer 14 in contact with the patient to form a seal around the wound.

After the dressing 10 is properly in position and adhered to a patient's skin, the support layer 16 can be removed. Generally removal of support layer 16 is accomplished by grasping the tab 20 and then using a peeling motion toward the edge of the dressing 10 to remove the support layer 16. Alternatively, tab 18 could be used to remove the support layer 16.

The wound dressing 10 of FIGs. 1 and 2 shows the backing material 14 shaped in the form of a cross, and the tab 20 takes the form of a generally rounded shape, however, as one skilled in the art should appreciate, the shape of the backing layer 14 and support layer 16 may take on various forms without departing from the spirit of the invention.

Turning now to FIGs. 3 and 4, a wound dressing 30 in accordance with a further embodiment of the present disclosure is shown. The wound dressing 30 as shown in FIG. 3, has a differently shaped tab portion 40, with the wound dressing 30 being similar to the dressing depicted in FIGs. 1 and 2 in other respects, with reference numbers referring to similar components of the wound dressings 10, 30.

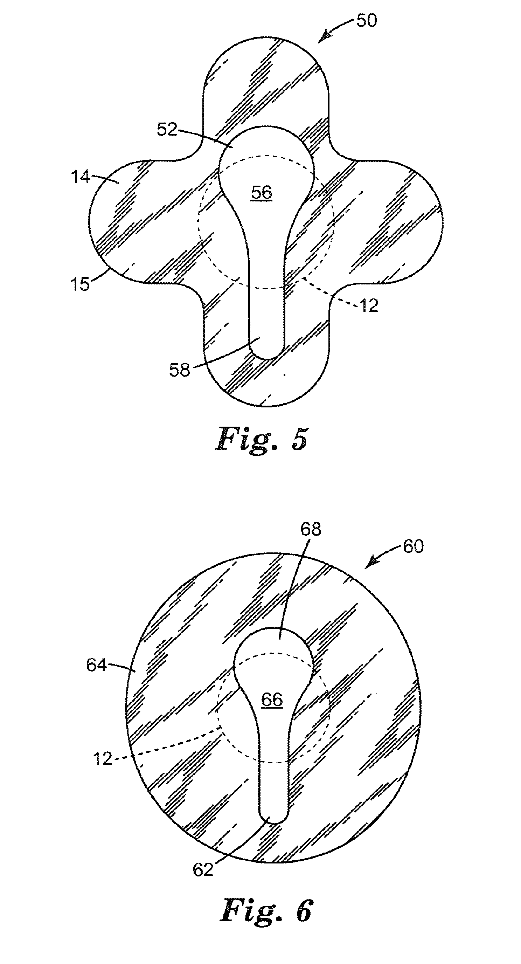

FIG. 5 depicts a wound dressing 50 in yet a further embodiment of the disclosure having a differently shaped support layer. Support layer 56 of wound dressing 50 has an end portion 58 that does not extend to the periphery of the wound dressing 50. Similar to the previously described embodiments, to facilitate handling of the wound dressing 50, the underside of the tab portion 52 is not attached to the backing 14 of the wound dressing (either by the use of a bond block material or other means). At least a portion of the remaining structure of the support layer 56, is removably attached to the surface of the backing layer 14, for example by heat bonding, or by the use of an adhesive.

FIG. 6 depicts yet a further design of a wound dressing 60 of the present disclosure. The wound dressing 60 depicted in FIG. 6 has a generally circular shaped backing 64, and a support layer 66 shaped similarly to the support layer 56 of FIG. 5, and having a tab portion 68, and an end portion 62. Similar to the other embodiments described herein, the support layer 66 is releasably adhered to a portion of the backing that overlaps a portion of the absorbent pad. For ease of handling, the tab portion 68 of the support layer 66 is not adhered to the backing 64, as described above. In addition, the tab portion 68 of the support layer 66 overlaps the absorbent pad 12 located on the opposite side of the backing 64 of the wound dressing 60.

In an embodiment, the wound dressing has a length (including the tabs) of approximately 8.5cm. In a further embodiment, such as the circular wound dressing shown in FIG. 6, the wound dressing has a diameter of approximately 7.5 cm.

In order to improve the conformability of the backing material on a highly contoured surface of a patient, the support layer should cover only a limited portion of the outer perimeter of the wound dressing. In one embodiment, the support layer 16, 56, 66 covers less than 40% of the perimeter of the backing, and in a further embodiment, the support layer covers less than 20% of the perimeter of the backing. In still a further embodiment, the support layer covers 0% of the perimeter of the backing, for example in the embodiments shown in FIGs. 5 and 6.

The wound dressings described herein are well suited for placement with one hand to highly contoured areas of the body such the toes or fingers. The placement of the tab opposite the absorbent pad, enables the user to easily position the dressing on a wound.

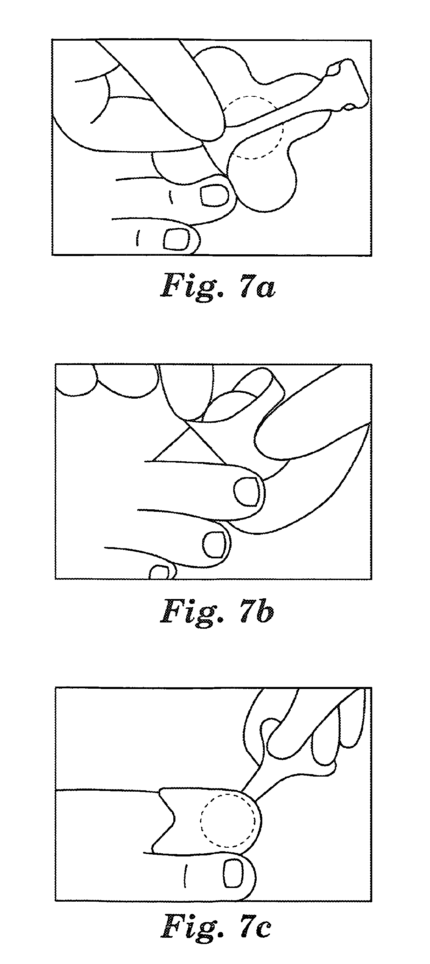

FIGs. 7a-c show the wound dressing of FIG. 3 being applied to a patient.
Once the wound and surrounding area have been cleaned, the user would first remove the release liner 24, if present, while holding the wound dressing by tab 40, as shown in FIG. 7a. As shown in FIG. 7b, the dressing is placed on the patient with the absorbent pad covering the wound area. The user would then apply slight hand pressure to the backing of the wound dressing in order to secure it in place. Finally, as shown in FIG. 7c, the support layer 16 is removed from the wound dressing by grasping tab 40 and peeling off the support layer 16.

When the support layer is removed from the backing layer 14, the force applied to the edge of the backing layer is generally perpendicular to the perimeter of the dressing 10. This force contrasts with peel forces of conventional support layers, which are parallel to the perimeter of the wound dressing, and which often lift the perimeter off the surface of the patient, potentially loosening the dressing or creating a path for leakage of fluids from the wound.

The absorbent pad 12 of dressing is sometimes referred to as an "island pad" because the backing layer extends substantially beyond the absorbent pad 12, typically beyond the entire periphery of the absorbent pad 12. For example, the diameter of the absorbent pad can be 2.5 cm, while a backing for this pad can be 7.2 cm by 7.2 cm.

The absorbent pad 12 can comprise a foam, a hydrocolloid or a hydrogel pad having a thickness of at least 1 mm (e.g., most preferably 3-9 mm), and the backing layer 14 can comprise a transparent elastic polymeric film (e.g., urethane) having a thickness no greater than 1 mm (e.g., most preferably 0.021-0.10 mm). It will be appreciated that in this embodiment the absorbent pad 12 is thicker, than the backing layer 14. The backing layer construction in this embodiment should be sufficiently stiff such that it will not fold over onto itself where it is not adequately supported by the support layer 16 or the absorbent pad 12. Typically, sufficient stiffness of the backing is obtained when at least a portion of the backing layer construction is comprised of a thickness greater than 0.070 mm. Other portions of the backing layer can be as thin as 0.021 mm (21 microns).

The support layer 16 is attached to the second major surface of the backing layer 14 (over the low adhesion backing). The bond between the support layer 16 and the backing layer 14 is stronger than the bond between the pressure sensitive adhesive 17 and the carrier layer 24 so that the backing layer 14 remains attached to the support layer 16 when the carrier layer 24 is removed from the dressing 10. A portion of the support layer is typically attached to the backing layer by heat seal bonding although other methods may be employed.

As one skilled in the art would appreciate, other implementations are appropriate in order to add or take away from the aspects the various embodiments of the wound dressings as described herein. For example, the backing layer 14 can be multiple films or coatings without diverging from the invention or deviating from the meaning of the term "film" as used herein. Similarly, the absorbent pad 12 can include multiple sub-layers, including films, webs, sheets, etc. Also, additional layers and films of other materials can be added between the materials described herein without deviating from the invention.

Additional aspects of various components of the invention will now be described in greater detail.

### Absorbent pad

Absorbent pad 12 can be manufactured of any of a variety of materials including, but not limited to, woven or nonwoven cotton or rayon. Absorbent pad 12 is useful for containing a number of substances, optionally including antimicrobial agents, drugs for transdermal drug delivery, chemical indicators to monitor hormones or other substances in a patient, etc.

The absorbent may include a hydrocolloid composition, including the hydrocolloid compositions described in U.S. Patent Nos. 5,622,711 and 5,633,010. The hydrocolloid absorbent may comprise, for example, a natural hydrocolloid, such as pectin, gelatin, or carboxymethylcellulose (CMC) (Aqualon Corp., Wilmington, Del.), a semi-synthetic hydrocolloid, such as cross-linked carboxymethylcellulose (X4ink CMC) (e.g. Ac-Di-Sol; FMC Corp., Philadelphia, Pa.), a synthetic hydrocolloid, such as cross-linked polyacrylic acid (PAA) (e.g., CARBOPOL^{™} No. 974P; B.F. Goodrich, Brecksville, Ohio), or a combination thereof. Generally, the hydrocolloid absorbent component comprises from about 5 percent to about 60 percent by weight of the absorbent composition. When preparing an absorbent composition for use in a wound dressing the hydrocolloid absorbent preferably comprises from about 20 percent to about 40 percent by weight of the composition. Absorbent materials may also be chosen from other synthetic and natural materials including polymer gels and foams.

### Backing Materials

Suitable backing materials for backing layer include, for example, nonwoven fibrous webs, woven fibrous webs, knits, films, porous films, and other familiar backing materials. The backing materials are typically translucent or transparent polymeric elastic films. The backing can be a high moisture vapor permeable film backing. U.S. Patent No. 3,645,835 describes methods of making such films and methods for testing their permeability. A combination of the aforementioned backings could also be used.

In an embodiment, the backing material is a polyurethane film. In a further embodiment, a polyurethane film backing laminated to a non-woven material is used. Such non-woven materials are described in U.S. Patent No. 5,088,483 (Heinecke) and U.S. Patent No. 6,881,875 (Swenson). The backing has a thickness of at least about 70 microns. In an embodiment, the thickness ranges from about 70 to about 500 microns. The backing layer construction in this embodiment should be sufficiently stiff such that it will not fold over onto itself where it is not adequately supported by the support layer 16 or the absorbent pad 12. Typically, sufficient stiffness of the backing is obtained when at least a portion of the backing layer construction is comprised of a thickness greater than 0.070 mm (70 microns).

The backing advantageously should transmit moisture vapor at a rate equal to or greater than human skin. In some embodiments, the adhesive coated backing layer transmits moisture vapor at a rate of at least 300 g/m²/24 hrs/37°C/100-10% RH, frequently at least 700 g/m²/24 hrs/37°C/100-10% RH, and most typically at least 2000 g/m²/24 hrs/37° C/100-10% RH using the inverted cup method.

The backing layer 14 is generally conformable to anatomical surfaces. As such, when the backing layer 14 is applied to an anatomical surface, it conforms to the surface even when the surface is moved. The backing layer 14 is also conformable to animal anatomical joints. When the joint is flexed and then returned to its unflexed position, the backing layer 14 can be made such that it stretches to accommodate the flexion of the joint, but is resilient enough to continue to conform to the joint when the joint is returned to its unflexed condition.

A description of this characteristic of backing layers 14 for use with the present invention can be found in issued U.S. Patent Nos. 5,088,483 and 5,160,315. Specific suitable backing materials are elastomeric polyurethane, co-polyester, or polyether block amide films. These films combine the desirable properties of resiliency, high moisture vapor permeability, and transparency found in backings.

### Support Layer

The material used to form the support layer 16 is generally more rigid than the backing layer 14 to provide adequate support to the backing layer and dressing 10 during application to a patient. The support layer 16 can be heat-sealable to the backing layer 14 with or without a low adhesion coating described above. In general, the support layer materials can include, but are not limited to, polyethylene/vinyl acetate copolymer-coated papers and polyester films. One example of a suitable support layer material is a polyethylene/vinyl acetate copolymer coated super calendared Kraft paper (1-80BKG-157 PE; Loparex of Willowbrook, Ill.). The support layer 16 can include a slit or perforations to form an area of weakness along a portion of the length of the support layer in order to facilitate folding of the dressing.

### Pressure Sensitive Adhesive

Various pressure sensitive adhesives can be used to form adhesive layer 17 on the backing layer 14 to make it adhesive. The pressure sensitive adhesive is usually reasonably skin compatible and "hypoallergenic", such as the acrylate copolymers described in U.S. Patent No. RE 24,906. Particularly useful is a 97:3 iso-octyl acrylate: acrylamide copolymer, as is 70:15:1 isooctyl acrylate: ethyleneoxide acrylate: acrylic acid terpolymer described in U.S. Patent No. 4,737,410. Additional useful adhesives are described in U.S. Patent Nos. 3,389,827, 4,112,213, 4,310,509, 4,323,557, WO 99/27975, and WO 99/28539. Inclusion of medicaments or antimicrobial agents in the adhesive is also contemplated, as described in U.S. Patent Nos. 4,310,509 and 4,323,557.

The pressure sensitive adhesive layer is generally provided on one major surface of the backing layer in order to make it adhesive, and a low adhesion coating (low adhesion backsize or LAB) is provided on the other major surface of the backing layer 14 on the side that comes in contact with the support layer. The low adhesion coating reduces the need to change the dressing due to unwanted dressing removal when other tapes or devices are placed on the dressing and removed, and reduces the surface friction of the dressing on linen or other fabrics, thereby offering additional protection against the accidental removal of dressing. A description of a low adhesion backing material suitable for use with the present invention can be found in U.S. Patent Nos. 5,531,855 and 6,264,976, which are compatible with a heat seal bond described below.

### Carrier Films

Carrier films 24 suitable for use with the invention can be made of kraft papers, polyethylene, polypropylene, polyester or composites of any of these materials. The films are preferably coated with release agents such as fluorochemicals or silicones. For example, U.S. Patent No. 4,472,480. describes low surface energy perfluorochemical liners. The liners are papers, polyolefin films, or polyester films coated with silicone release materials. Examples of commercially available silicone coated release papers are POLYSLIK™, silicone release papers available from Rexam Release (Bedford Park, Ill.) and silicone release papers supplied by Loparex Inc. (Willowbrook, Ill.).

As various changes could be made in the above constructions, compositions and methods without departing from the scope of the invention as defined in the claims, it is intended that all matter contained in the above description or shown in the accompanying drawings be interpreted as illustrative and not in a limiting sense.

## Claims

1. A wound dressing (10) and delivery system comprising:
a wound dressing (10) comprising a backing layer (14) comprising a transparent elastic film, the backing layer having a first and second major surface; wherein at least a portion of the backing layer (14) has a thickness of at least about 70 microns;
an adhesive layer (17) on at least a portion of the first major surface of the backing layer (14);
a dressing support layer (16) releasably adhered to the second major surface of the backing layer (14), the dressing support layer (16) having a tab portion (20) which overlaps at least a portion of the backing layer (14); and
an absorbent pad (12) comprising a foam, a hydrocolloid, or a hydrogel pad having a thickness of at least 1 mm.

2. The wound dressing and delivery system of claim 1, wherein the support layer is releasably adhered to a portion of the second major surface which overlaps a portion of the absorbent pad.

3. The wound dressing of claim 1 or 2, wherein the tab portion is not adhered to the backing layer.

4. The wound dressing (10) and delivery system of claim 1, wherein the dressing support layer (16) has a substantially linear configuration with the tab portion (20) at one end and an end portion (18) opposite the tab portion (20), wherein the end portion (18) extends to only a portion of a perimeter (15) of the backing layer (14).

5. The wound dressing (10) and delivery system of claim 1, wherein the dressing support layer (16) has a substantially linear configuration with the tab portion (20) at one end and an end portion (18) opposite the tab portion (20), wherein the end portion (18) does not extend to a perimeter (15) of the backing layer (14).

6. The wound dressing and delivery system of claims 4 or 5, wherein the support layer is releasably attached to the portion of the second major surface of the backing layer that overlaps a portion of the absorbent pad.

7. The wound dressing of claims 4 or 5, wherein the end portion covers less than 40% of the perimeter of the backing layer.

## Patentansprüche

1. Wundverband- (10) und Abgabesystem, umfassend:
einen Wundverband (10), der eine Rückenschicht (14) umfasst, welche eine transparente, elastische Folie aufweist, wobei die Rückenschicht eine erste und zweite Hauptfläche aufweist; wobei mindestens ein Abschnitt der Rückenschicht (14) eine Dicke von mindestens ungefähr 70 Mikrometer aufweist;
eine Klebeschicht (17) an mindestens einem Abschnitt der ersten Hauptfläche der Rückenschicht (14);
eine Verbandsträgerschicht (16), die ablösbar an der zweiten Hauptfläche der Rückenschicht (14) haftet, wobei die Verbandsträgerschicht (16) einen Streifenabschnitt (20) aufweist, der mit mindestens einem Abschnitt der Rückenschicht (14) überlappt; und
ein saugfähiges Kissen (12), das ein Schaumstoff-, ein Hydrokolloid- oder ein Hydrogelkissen mit einer Dicke von mindestens 1 mm umfasst.

2. Wundverband- und Abgabesystem nach Anspruch 1, wobei die Trägerschicht ablösbar an einem Abschnitt der zweiten Hauptfläche haftet, die einen Abschnitt des saugfähigen Kissens überlappt.

3. Wundverband nach Anspruch 1 oder 2, wobei der Streifenabschnitt nicht an der Rückenschicht anhaftet.

4. Wundverband- (10) und Abgabesystem nach Anspruch 1, wobei die Verbandsträgerschicht (16) eine im Wesentlichen geradlinige Konfiguration aufweist, mit dem Streifenabschnitt (20) an einem Ende und einem Endabschnitt (18) gegenüber dem Streifenabschnitt (20), wobei der Endabschnitt (18) sich lediglich zu einem Abschnitt eines Umfangs (15) der Rückenschicht (14) erstreckt.

5. Wundverband- (10) und Abgabesystem nach Anspruch 1, wobei die Verbandsträgerschicht (16) eine im Wesentlichen geradlinige Konfiguration aufweist, mit dem Streifenabschnitt (20) an einem Ende und einem Endabschnitt (18) gegenüber dem Streifenabschnitt (20), wobei der Endabschnitt (18) sich nicht zu einem Umfang (15) der Rückenschicht (14) erstreckt.

6. Wundverband- und Abgabesystem nach Anspruch 4 oder 5, wobei die Trägerschicht ablösbar an dem Abschnitt der zweiten Hauptfläche der Rückenschicht angebracht ist, die einen Abschnitt des saugfähigen Kissens überlappt.

7. Wundverband nach Anspruch 4 oder 5, wobei der Endabschnitt weniger als 40 % des Umfangs der Rückenschicht überdeckt.

## Revendications

1. Pansement (10) et système d'application comprenant :
un pansement (10) comprenant une couche dorsale (14) comprenant un film élastique transparent, la couche dorsale comportant une première surface principale et une deuxième surface principale ; dans lequel au moins une partie de la couche dorsale (14) a une épaisseur d'au moins environ 70 microns ;
une couche adhésive (17) sur au moins une partie de la première surface principale de la couche dorsale (14) ;
une couche de support de pansement (16), collée de façon amovible sur la deuxième surface principale de la couche dorsale (14), la couche de support du pansement (16) comportant une partie constituée d'une languette (20) qui chevauche au moins une partie de la couche dorsale (14) ; et
un matelas absorbant (12) comprenant un matelas constitué d'une mousse, d'un hydrocolloïde ou d'un hydrogel ayant une épaisseur d'au moins 1 mm.

2. Pansement et système d'application selon la revendication 1, dans lesquels la couche de support est collée de façon amovible sur une partie de la deuxième surface principale qui chevauche une partie du matelas absorbant.

3. Pansement selon la revendication 1 ou 2, dans lequel la partie constituée d'une languette n'est pas collée sur la couche dorsale.

4. Pansement (10) et système d'application selon la revendication 1, dans lesquels la couche de support du pansement (16) a une configuration sensiblement linéaire, la partie constituée d'une languette (20) étant située à une extrémité et une partie terminale (18) se trouvant à l'opposé de la partie constituée d'une languette (20), la partie terminale (18) s'étendant jusqu'à une partie seulement d'un périmètre (15) de la couche dorsale (14).

5. Pansement (10) et système d'application selon la revendication 1, dans lesquels la couche de support du pansement (16) a une configuration sensiblement linéaire, la partie constituée d'une languette (20) étant située à une extrémité et une partie terminale (18) se trouvant à l'opposé de la partie constituée d'une languette (20), la partie terminale (18) ne s'étendant pas jusqu'à un périmètre (15) de la couche dorsale (14).

6. Pansement et système d'application selon la revendication 4 ou 5, dans lesquels la couche de support est fixée de façon amovible à la partie de la deuxième surface principale de la couche dorsale qui chevauche une partie du matelas absorbant.

7. Pansement selon la revendication 4 ou 5, dans lequel la partie terminale couvre moins de 40 % du périmètre de la couche dorsale.
